# NEW EUROPEAN PATENT SPECIFICATION

(11) **EP 1 343 809 B2**
(45) Date of publication and mention of the opposition decision: **15.03.2023**
(45) Mention of the grant of the patent: 24.06.2015
(21) Application number: 00992709.6
(22) Date of filing: 14.12.2000
(51) Int. Cl.: C07K 1/16, C07K 1/18, C07K 1/30, C07K 14/81

(54) **METHOD OF PREPARING ALPHA-1 PROTEINASE INHIBITOR**
VERFAHREN ZUR HERSTELLUNG EINES ALPHA-1-PROTEINASEINHIBITORS
PROCEDE POUR L'ELABORATION D'UN INHIBITEUR DE PROTEINASE ALPHA-1

(43) Date of publication of application: 17.09.2003
(73) Proprietor: Grifols Therapeutics Inc., Research Triangle Park, NC 27709 (US)
(72) Inventor: LEBING, Wytold, Clayton, NC 27520 (US); CHAVEZ, Mark, D., Raleigh, NC 27613 (US); OWNBY, David, W., Clayton, NC 27520 (US); TRUKAWINSKI, Susan, Cary, NC 27511 (US); WOOD, Woody, D., Pine Level, NC 27568 (US)
(74) Representative: Durán Moya, Luis-Alfonso
(86) International application number: PCT/US2000/042811
(87) International publication number: WO 2002/048176

(56) References cited:
- EP-A1- 0 698 615
- WO-A1-95/35306
- WO-A1-97/09350
- US-A- 4 379 087
- US-A- 4 656 254
- LAEGREID W W ET AL: "ISOLATION AND SOME PROPERTIES OF EQUINE ALPHA-1 ANTI TRYPSIN" INTERNATIONAL JOURNAL OF BIOCHEMISTRY, vol. 14, no. 4, 1982, pages 327-334, XP002285196 ISSN: 0020-711X
- HEIN R H ET AL: "Production of alpha 1-proteinase inhibitor (human)." THE EUROPEAN RESPIRATORY JOURNAL. SUPPLEMENT. DENMARK MAR 1990, vol. 9, March 1990 (1990-03), pages 16s-20s, XP009032419 ISSN: 0904-1850
- KUHN CHERYL R ET AL: "The isolation and partial characterization of alpha-1-proteinase inhibitor from the serum of the ostrich (Struthio camelus)" INTERNATIONAL JOURNAL OF BIOCHEMISTRY, vol. 26, no. 6, 1994, pages 843-853, XP002285197 ISSN: 0020-711X
- CHEN ET AL.: 'Purification of alpha1 proteinase inhibitor from human plasma fration IV-1 by ion exchange chromatogrpahy' VOX SANG vol. 74, 1998, pages 232 - 241, XP002940189
- CHEN ET AL.: 'Chromatographic purification of human alpha1 proteinase inhibitor from dissolved cohn fration IV-1 paste' J. CHROMATOGRAPHY vol. A800, 1998, pages 207 - 218, XP002940190
- COAN ET AL.: 'Preparation and properties of alpha1-proteinase inhibitor concentrate from human plasma' VOX SANG vol. 48, 1985, pages 333 - 342, XP002940191

## Description

### Field of the Invention

The invention relates to the purification of alpha-1 proteinase inhibitor (α-1 PI) from aqueous solutions. More specifically, the invention relates to the purification of α-1 PI from blood plasma or from plasma fractions produced from Cohn-Oncley fractionation with chromatography. Viral removal is accomplished by the addition of PEG and by nanofiltration. Inactivation of enveloped viruses is accomplished by addition of detergent prior to the chromatography process.

### Background

Alpha-1 proteinase inhibitor (α-1 PI) is a glycoprotein with a molecular weight of about 55,000 Daltons. The protein is a single polypeptide chain to which several oligosaccharide units are covalently bound. Alpha-1 PI acts as an inhibitor of endogenous proteases, such as trypsin, chymotrypsin, pancreatic elastase, skin collagenase, renin, urokinase and proteases of polymorphonuclear lymphocytes.

Alpha-1 PI is currently used therapeutically to treat persons having a genetically caused deficiency of α-1 PI. In such a condition, α-1 PI is administered to inhibit lymphocyte elastase in the lungs. Lymphocyte elastase breaks down foreign proteins in the lungs. When α-1 PI is not present in sufficient quantities to regulate elastase activitiy, the elastase breaks down lung tissue. In time, this imbalance results in chronic lung tissue damage and emphysema. Alpha-1 PI has been successfully used to treat this form of emphysema.

The demand for α-1 PI typically exceeds the available supply. Alpha-1 PI for therapeutic use is currently purified from human plasma. This source of the protein is limited, which contributes to the low supply. In order to maximize the available supply of α-1 PI, a process for purifying α-1 PI from human plasma should have the highest possible yield. The purity of the α-1 PI isolated from human plasma is also critical, because trace impurities can stimulate immune responses in patients who are receiving a 1 PI. Finally, the process of purifying α-1 PI from human plasma using current techniques requires an extensive amount of time, for the separation of the α-1 PI from other proteins, viruses, etc.. All of these factors (i. e., low yields, long production times, and low purity), contribute to the inadequate supply of α-1 PI.

Various methods of purifying α-1 PI from human plasma have been described. Bollen, et al., U. S. Pat. No. 4,629,567 (1986) used five different chromatography steps to purify the α-1 PI from yeast, *E. coli,* and human plasma. The five steps involved DEAE ion exchange, thiol-disulfide exchange, heparin affinity, zinc-chelate chromatography, and amino hexyl ion exchange. No purity and yield data were shown.

Novika, et al., Gematol. Transfuziol. 34: 46-50 (1989) reported isolation methods from the by-products of the manufacture of blood products. They used affinity, DEAE cellulose, and gel filtration chromatographies. The purity and yield data were not available.

Podiarene, et al., Vopr. Med. Khim. 35: 96-99 (1989) reported a single step procedure for isolation of α-1 PI from human plasma using affinity chromatography with monoclonal antibodies. Alpha-1 PI activity was increased 61.1 fold with a yield of 20%.

Burnouf, et al., Vox. Sang. 52,291-297 (1987) starting with plasma supernatant A (equivalent to Cohn Fraction II + III) used DEAE chromatography and size exclusion chromatography to produce an α-1 PI which was 80-90% pure (by SDS-PAGE) with a 36-fold increase in purity. Recovery was 65-70% from the supernatant A.

Hein, et al., Eur. Respir. J. 9: 16s-20s (1990) and co-owned U. S. Patent No. 4,697,003 present a process which employs Cohn Fraction IV-1 as the starting material and utilizes fractional precipitation of α-1 PI with polyethylene glycol followed by anion exchange chromatography on DEAE Sepharose^{®}. The final product has a purity of about 60% with 45% yield.

Dubin, et al., Prep. Biochem. 20: 63-70 (1990) have shown a two step chromatographic purification. First α-1 PI, CI inhibitor, α-1 antichymotrypsin, and inter α-1 trypsin inhibitor were eluted from Blue Sepharose@ and then α-1 PI was purified by gel filtration. Purity and yield data were not available.

Ballieux, *et al*., purified an α-1 PI and proteinase-3 complex from purulent sputum using 4-phenylbutylamine affinity chromatography, cation exchange, and a final immunoaffinity step (Ballieux, B. E., et al., J. Immunol. Methods 159: 63-70 (1993)). The pH of the buffer used in the cation exchange step was 7.0. Under the conditions used, most of the sputum proteins bound to the resin, but α-1 PI and proteinase-3 passed through without binding.

Jordan, et al., U. S. Pat. No. 4,749,783 (1988) described a method where biologically inactive proteins in a preparation were removed by affinity chromatography after a viral inactivation step. The basis of the separation between the native and denatured forms of the protein was the biological activity of the native protein towards the affinity resin and not physical differences between the native and denatured proteins.

Lebing and Chen, co-owned U. S. Pat No. 5,610,285, described a method where α-1 PI was captured from IV-1 paste suspension using a DEAE chromatography step. The collected solution was ultrafiltered then applied to an S-cation column for initial purification. Alpha-1 PI was collected as the flow-through fraction. The product, in a sucrose solution, was then treated with TNBP/cholate in order to inactivate viruses. Following filtration and ultrafiltration, the product was applied to a second S-cation column for final purification. Once the product was formulated and freeze dried, it was virally inactivated a second time by heating to 80 C for 72 hours. Product purity and virus safety were greatly improved versus the Hein process, described above, but, in practice, the Lebing and Chen process was too resource intensive for large-scale manufacturing.

Coan et al. (U. S. Pat No. 4379087A) described a method for separating alpha-1 PI from blood plasma or blood plasma fractions which contain PI. Blood plasma is fractionated to give a Cohn fraction containing PI. An aqueous solution of the PI-containing Cohn fraction is prepared, and the pH of the solution is adjusted to about 6.5-8.5 After a hold period of 0.2-24h at 2°-50°, a polycondensed polyglycol, for example, polyethylene glycol (PEG) is added to the solution, the pH of which is then adjusted to about 4.6-5.9 to precipitate unwanted proteins from the effluent in which a proportion of the PI is retained. The effluent is treated to separate PI therefrom directly or with further purification.

The U. S. Pat No. 6093804 discloses the separation of active from inactive Alpha Proteinase Inhibitor (API) in a single separation process on an anion exchange column. Further purification can be achieved by passing the eluate of the anion exchange step through a cation exchange resin. The method involves generally the removal of lipoproteins from an initial protein suspension by use of a lipid removal agent, followed by successive separations with anon and cation exchange resins tiwh specific eluants.

A process for the purification of α-1 PI that improves the yield and purity of the α-1 PI, that requires a shorter production time and uses less resources (such as reagents, water, resins, and column size) is needed. The present invention provides a process of purifying α-1 PI from a blood plasma fraction with a higher yield, higher purity, shorter production time, and use of less resources than known methods.

### Summary of the Invention

The present invention provides method for purifying α-1 proteinase inhibitor from an aqueous solution containing α-1 proteinase inhibitor, comprising the steps of :
(a) removing a portion of contaminating proteins from the aqueous solution to obtain a purified solution containing α-1 proteinase inhibitor, wherein said removing step comprises the steps of
   (i) precipitating said portion of contaminating proteins from said aqueous solution by adding a polyethylene glycol with a MW between about 3,000 and about 4000 to said aqueous solution at a concentration between about 3% to 15% weight per volume, and adjusting the pH of said aqueous solution from about 5.0 to about 6.0; and
   (ii) separating said precipitated portion of contaminating proteins from said aqueous solution, thereby obtaining said purified solution containing α-1 proteinase inhibitor; then
(b) inactivating viruses prior to diluting said purified solution to reduce the conductivity of said purified solution so that α-1 proteinase inhibitor binds to an anion exchange resin, wherein said viral inactivation step comprises the steps of (a) adding a detergent to said purified solution to obtain a mixture of detergent and purified solution; and (b) adjusting the pH of said mixture to from about 6.5 to about 8.5, and wherein said detergent is a non-ionic detergent, and wherein said non-ionic detergent is Tween 20; then
(c) passing said purified solution through an anion exchange resin so that α-1 proteinase inhibitor binds to said anion exchange resin; then
(d) eluting α-1 proteinase inhibitor from said anion exchange resin to obtain an eluted solution containing α-1 proteinase inhibitor; then
(e) adjusting the pH, conductivity and protein concentration of said eluted solution containing α-1 proteinase inhibitor so that α-1 proteinase inhibitor does not bind to a cation exchange resin;
(f) passing the eluted solution through a cation exchange resin; and
(g) collecting a flow-through from said cation exchange resin that contains α-1 proteinase inhibitor.

The present invention also provides a method for purifying α-1 proteinase inhibitor from Cohn Fraction IV-1, comprising the steps of :
(a) removing a portion of contaminating proteins from the Cohn Fraction IV-1 in order to obtain a purified solution containing α-1 proteinase inhibitor, wherein said removing step comprises the steps of
   (i) precipitating said portion of contaminating proteins from said Cohn Fraction IV-1 by adding a polyethylene glycol with a MW between about 3,000 and about 4000 to said Cohn Fraction IV-1 at a concentration between about 3% to 15% weight per volume, and adjusting the pH of said Cohn Fraction IV-1 from about 5.0 to about 6.0; and
   (ii) separating said precipitated portion of contaminating proteins from said Cohn Fraction IV-1, thereby obtaining said purified solution containing α-1 proteinase inhibitor; then
(b) inactivating viruses prior to adjusting the conductivity of said purified solution so that α-1 proteinase inhibitor binds to an anion exchange resin, wherein said viral inactivation step comprises the steps of (a) adding a detergent to said purified solution to obtain a mixture of detergent and purified solution; and (b) adjusting the pH of said mixture to from about 6.5 to about 8.5, and wherein said detergent is a non-ionic detergent, and wherein said non-ionic detergent is Tween 20; then
(c) passing said purified solution through the anion exchange resin so that α-1 proteinase inhibitor binds to said anion exchange resin; and then
(d) eluting α-1 proteinase inhibitor from said anion exchange resin to obtain an eluted solution containing α-1 proteinase inhibitor; then
(e) adjusting the pH, conductivity and protein concentration of said eluted solution containing α-1 proteinase inhibitor so that α-1 proteinase inhibitor does not bind to a cation exchange resin;
(f) passing the eluted solution through a cation exchange resin; and
(g) collecting a flow-through from said cation exchange resin that contains α-1 proteinase inhibitor.

In another embodiment of the invention, said diluting step (b) or adjusting step (b) comprises diluting said purified solution so that said purified solution has a conductivity of between about 2.0 mS and about 6.0 mS. In this embodiment the purified solution can be diluted with water, wherein the water can contain sodium phosphate.

In another embodiment of the invention, the step of adjusting a pH of said purified solution to between about 6.25 and about 7.25 prior to passing said purified solution through the anion exchange resin.

In another embodiment of the invention, the Cohn Fraction IV-1 is suspended in an aqueous solution.

### Detailed Description

The present invention provides a process for purifying α-1 PI from an aqueous solution containing α-1 PI, such as human plasma, for example. In methods of the invention, contaminating proteins are removed from the aqueous solution before it is passed through an anion exchange resin. Prior to passing the solution through the anion exchange resin, the conductivity of the solution is adjusted, preferably by diluting the solution, so that the α-1 PI will bind to the anion exchange resin. This typically occurs at a conductivity of between about 2.0 mS and about 6.0 mS. The α-1 PI is then selectively eluted from the anion exchange resin to provide α-1 PI at yield and purity levels above those obtained with current processes.

A known procedure for the purification of α-1 PI begins with Fraction IV-1 paste, as obtained through the Cohn-Oncley fractionation procedure for human plasma. See, e. g., E. J. Cohn, et al., J. Amer. Chem. Soc., 68,459 (1946); E. J. Cohn, U. S. Patent No. 2,390,074; and Oncley, et al., J. Amer. Chem. Soc., 71,541 (1949) the entire disclosures of which are hereby incorporated by reference herein. The Cohn-Oncley process involves a series of cold ethanol precipitation steps during which specific proteins are separated according to isoelectric point by adjusting pH, ionic strength, protein concentration, temperature and ethanol concentration. The Fraction IV-1 paste obtained by this procedure is dissolved in a buffer solution and heated to activate α-1 PI. An initial purification step includes the precipitation of contaminating proteins and lipids from the dissolved Fraction IV-1. The α-1 PI is then precipitated from the dissolved Fraction IV-1 solution, and the crude α-1 PI is passed through an anion exchange resin to remove contaminating proteins. A viral inactivation is accomplished by pasteurization for 10 hours at 60 C. in a sucrose solution. Following pasteurization, the α-1 PI is diafiltered, bulked in NaCl/Na₃PO₄, sterile filtered, and lyophilized.

In one embodiment of the invention, the starting material for the process is Cohn Fraction IV-1, although other fractions, such as Cohn Fraction II + III, for example, can be used as a starting material. This fraction may be dissolved in an aqueous solution, such as a tris- (hydroxymethyl) amino methane (Tris) buffer solution, for example.

Fraction IV-1 is typically a paste that can be dissolved in Tris buffer at a pH of about 9.25 to about 9.5 at about 40 C. A salt, such as sodium chloride (NaCl) may also be added to the solution.

The process of the invention includes the removal of at least a portion of contaminating proteins from the aqueous solution to obtain a purified solution that contains α-1 PI. Such contaminating proteins may include fibrinogen and albumin, for example. The portion of contaminating proteins is preferably removed by precipitation with a polyalkylene glycol, such as polyethylene glycol (PEG) or polypropylene glycol (PPG), for example. Other alcohols that are known to those of skill in the art to have similar properties may be used. PEG, the preferred polyalkylene glycol for use in methods of the invention, has a molecular weight of between about 2,000 and about 10,000, and preferably has a molecular weight of between about 3,000 and about 4,000. The PEG added to the solution is at least about 2% weight per volume of the mixture formed, is preferably about 3% to 15%, and is most preferably 11.5%. The pH of the solution may also be adjusted to precipitate the contaminating proteins. The pH is typically adjusted to between about 5.0 and about 6.0. The pH of the solution is adjusted by addition of an acid, such as acetic acid. The precipitate may then be separated from the solution by filtration, centrifugation, or any other conventional means known in the art, to obtain a filtrate containing α-1 PI.

The conductivity of the filtrate is then adjusted prior to passing the filtrate over an anion exchange resin. The equilibrium between an ion exchange resin and a protein solution is influenced by the ionic strength of the solution (see, e. g., Yamamato, et al., Biotechnol. Bioeng., 25: 1373-91 (1983)). The conductivity of the filtrate is therefore adjusted so that the α-1 PI in the filtrate will bind to an anion exchange resin. This conductivity is typically between about 2.0 mS and 6.0 mS when measured at 25 C., but other ranges of conductivity may be necessary to bind the α-1 PI to an anion exchange resin. The conductivity is preferably adjusted by dilution of the filtrate, and not by gel filtration, diafiltration, or other means of salt removal. The filtrate is preferably diluted with water, which may contain sodium phosphate (Na₃PO₄), or other buffers capable of providing a pH of about 6-7.

After dilution of the filtrate, the solution is applied directly to an anion exchange resin. Unlike known methods, as described above, the filtrate is not subjected to further PEG precipitation or diafiltration prior to chromatographic separation. The diluted filtrate is passed over an anion exchange resin, which is preferably a quaternary aminoethyl (QAE) resin. While QAE chromatography is preferred, other anion exchange resins, such as trimethylamino ethane (TMAE) and diethyl aminoethyl (DEAE), may be used in methods of the invention. The α-1 PI binds to the anion exchange resin. In a preferred embodiment, the anion exchange resin is washed with a buffer solution, such as an Na₃PO₄ buffer, to remove another portion of contaminating proteins. The proteins typically removed are albumin and transferrin. During the buffer wash, α-1 PI remains bound to the anion exchange resin. After the buffer wash, α-1 PI is eluted from the anion exchange resin to obtain an eluted solution containing purified α-1 PI. Ceruloplasmin remains bound to the column during both the wash and elution.

A further purification of the protein may be accomplished by passing the eluted solution containing the α-1 PI through a cation exchange resin. The pH, conductivity, and protein concentration of the eluted solution are adjusted so. that α-1 PI does not bind to the cation exchange resin. The influences of pH, conductivity, and protein concentration on the binding of α-1 PI to a cation exchange resin are set forth in coowned U. S. Patent No. 5,610,825, the entire disclosure of which is hereby incorporated by reference herein.

Viral inactivation and/or viral removal also play a part in the purification of α-1 PI from aqueous solutions, such as human plasma, for example. Known processes for the purification of α-1 PI utilize a dry heat treatment for the inactivation of viruses. This treatment can denature α-1 PI protein, however, thereby reducing the yield and/or purity of the α-1 PI. The methods of the invention deactivate and remove viruses without this heat treatment step, thereby increasing both yield and purity of α-1 PI obtained.

The above-described precipitation of contaminating proteins with 11.5% PEG also serves as one of the virus removal steps. Precipitation with 11.5% PEG removes both enveloped and non-enveloped viruses from the blood plasma fraction. This precipitation removes, with a : 4 logs of clearance, at least four viruses, including HIV-1, BVDV, PRV, and Reovirus Type 3. In comparison, the dry heat process of known methods only results in > 4 logs of clearance of three of these viruses; the Reovirus Type 3 is only removed at 1 log clearance. Additionally, the 11.5% PEG precipitation step has been shown to result in > 4 logs of clearance of transmissible spongiform encephalopathies (i. e., TSE prions) from the blood plasma fraction. (See co-owned, copending application entitled Method of Separating Prions from Biological Material, filed on even-date herewith.)

Another viral deactivation is accomplished by addition of a non-ionic detergent to the aqueous solution. This step is taken prior to passing the solution through the anion exchange resin. Non-ionic detergents for use in methods of the invention Tween 20. **Preferably,** Tween 20 is added -at from -about 0.33% to about 10% weight per volume of resulting mixture. Tween 20 is preferably added in the range of about 0.5% to about 2.0% and is most preferably added at 1.0%. The detergent treatment with 1% Tween 20 reduces enveloped viruses by > 4 logs of clearance.

Another embodiment of the invention includes virus removal. Both enveloped and non-enveloped viruses are removed by filtration, preferably by nanofiltration, or any other filtration methods known in the art. In a preferred embodiment, the solution eluted from the ion exchange resin, which includes α-1 PI, is subjected to nanofiltration. Nanofiltration reduces both enveloped and non-enveloped viruses by > 4 logs of clearance.

The methods of the current invention, therefore, preferably include two > 4 logs of clearance steps for the removal of enveloped viruses and non-enveloped viruses. These viral clearance levels are greater than those obtained by known methods of manufacture of α-1 PI, which only include two virus clearance steps.

Practice of the invention will be understood more fully from the following examples, which are presented herein for illustration only and should not be construed as limiting the invention in any way.

### Example 1

In a preferred embodiment, the starting material is Cohn Fraction IV-1 paste, which is obtained by the Cohn-Oncley fractionation technique, well known to those of skill in the art. The preparation of an aqueous solution from the Fraction IV-1 paste is described below.

The IV-1 paste is dissolved in 24 volumes of Tris buffer (IV-1 paste weight in kg times 24) between 2'and 8'C. The solution is mixed for approximately 4.5 hrs. while maintaining the temperature between 2'and 8'C. After mixing, the pH of the solution is adjusted to between 9.25 and 9.5 using 1.0 M NaOH, which is added at a rate of 1.25 1/min. This solution is then mixed for 1 hr. and the pH readjusted, if necessary. The solution is then heated to 39'to 41'C. for 60 to 90 minutes to dissolve the Fraction IV-1 paste in the buffer solution.

### Example 2

Fraction IV-1, like other plasma fractions, contains various proteins, such as lipoproteins, immunoglobulins, globulin, metaprotein, etc. These proteins must be separated from the α-1 PI, but some will also bind to an ion exchange resin and thereby interfere with the purification of α-1 PI. Before adding the solution to an anion exchange resin, therefore, a portion of these contaminating proteins is preferably removed first. This example describes one purification step in the process for the removal of contaminating proteins.

The Cohn Fraction IV-1 dissolved in the Tris buffer solution, as described above, is again cooled to between 2 and 8 C. To this cooled solution is added NaCl to 0.11 M. To this solution is then added 11.5% PEG MW 3,350 (Carbowax, Union Carbide, Danbury, CT; suspension weight in kg times 0.115). The pH of the solution is then adjusted to between 5.10 and 5.35 with 1.0 M glacial acetic acid. To this is added 2.5% of Hyflo Supercel (Celite Corporation, Lompoc, CA) and the resulting solution is mixed for 10 min. A precipitate of contaminating proteins and viruses, including prion proteins, is formed. This precipitate is filtered through a filter press assembled with CP90 Cuno filter pads (Cuno, Meriden, CT) at NMT 20 psi. The press is rinsed with 11.5% PEG in a water buffer. The paste obtained by the filtration is discarded. The filtrate contains the α-1 PI in PEG. Alternatively, the precipitate may be centrifuged and then filtered.

### Example 3

In a preferred embodiment, the filtrate obtained from the PEG precipitation outlined in Example 2 above is subjected to viral inactivation in a non-ionic detergent. The pH of the filtrate from Example 2 above is adjusted to 7.0 to 7.2 with 1.0 M NaOH. To this solution is added Tween 20 to 1 % (PEG filtrate weight in kg times 10.1 g/kg), and the pH adjusted to 6.9 to 7.1 with 1.0 M NaOH. This solution is held between 20 and 30 C. for 8-10 hrs. This treatment reduces enveloped viruses in the solution containing α-1 PI by > 4 logs of clearance.

### Example 4

The solution containing α-1 PI in both PEG and Tween 20 is then passed through an anion exchange resin to further purify the α-1 PI and separate it from the PEG and Tween 20. Prior to addition of the solution to the anion exchange resin, the conductivity of the solution is adjusted so that the α-1 PI will bind to the anion exchange resin. The solution resulting from Example 3 above is diluted with water-for-injection (WFI) until the conductivity of the solution is reduced to a value between about 2.0 mS and about 6.0 mS at 25 C. Additional 1% Tween 20 may be included in the WFI to prolong the contact time of the solution with the detergent. The WFI may contain Na₃PO₄ (20 mM) at a pH of 6. 5.

A Q Sepharose^{®} (Amersham-Pharmacia Biotech, Upsala, Sweden) fast flow column is prepared and equilibrated with a 20 mM Na3PO4 solution at pH 6.5. The solution of α-1 PI in Tween 20 and PEG is then added to the column at a concentration of 8-12 mg of α-1 PI per milliliter of resin. The flow rate of the column is 125 cm/hr. The α-1 PI binds to the column, which is then washed with the 20 mM Na₃PO₄ solution at pH 6.5. The Na₃PO₄ buffer further removes contaminating proteins, such as albumin and transferrin, for example, from the column. An elution buffer of 0.025 M Na₃PO₄/0.1 M NaCl at pH 6.95-7.05 is passed through the column to remove the α-1 PI. The eluate, which contains the α-1 PI, is collected. Ceruloplasmin remains bound to the column until the NaCl strip.

This purification step, therefore, accomplishes four objectives: 1) separation of the α-1 PI from the PEG; 2) separation of the α-1 PI from the Tween 20; 3) purification of the α-1 PI; and 4) prolongation of the contact time of viruses in the solution with the Tween 20.

### Example 5

In a preferred embodiment of the invention, the aqueous solution containing α-1 PI is subjected to a further purification step following the Q Sepharose chromatography outlined in Example 4 above. The further purification is accomplished by cation chromatography.

A Macro Prep High SO (BioRad Laboratories, Hercules, CA) column is prepared and equilibrated with a 20 mM Na₃PO₄/5 mM NaCl buffer at pH 5.45 to 5.54 until the column effluent consistently has a pH < 5.60. Prior to adding the eluate containing α-1 PI obtained from the method of Example 4 above to the cation column, it may be concentrated by ultrafiltration and diafiltration. Dry Na₃PO₄ and NaCl are then added to the resulting concentrate to a final concentration of 20 mM Na₃PO₄ and 5 mM NaCl. The pH of the resulting solution is then adjusted to approximately 5.50 with 1.0 M glacial acetic acid. This solution is then applied to the column at a ratio of 5 mg contaminants (e. g., albumin and IgA) per milliliter of resin. The α-1 PI does not bind to the resin, while the contaminants do bind to the resin. The α-1 PI is chased through the column with a 20 mM Na₃PO₄/5 mM NaCl buffer at pH 5.45 to 5.54 to obtain a solution containing a-I PI.

### Example 6

To further remove contaminating viruses, a filtration step is preferably included in methods of the invention. To the solution containing α-1 PI obtained by the process of Example 5 above is added NaCl to 0.75 M and the pH is adjusted to 7.0 with NaOH. The solution is then concentrated on a Viresolve 70 (D (Millipore, Bedford, MA) membrane using differential diafiltration with 0.75 M NaCl until the volume is reduced to 5%-20% of its original volume. Alpha-1 PI is washed through the Viresolve 7000 membrane with 3-5 diafiltration volumes of 0.75 M NaCl.

Following the filtration, the resulting solution containing purified α-1 PI is concentrated by ultrafiltration and diafiltration. After diafiltration, the solution is concentrated, and the concentrated α-1 PI is formulated at about 55 mg of α-1 PI per milliliter of 20 mM Na₃PO₄ and 100 mM NaCl at pH 7.0. The formulated solution is sterile filtered. The resulting solution is lyophilized using methods known in the art.

The invention may be embodied in other specific forms without departing from the spirit or essential characteristics thereof. The foregoing examples are included by way of illustration only. Accordingly, the scope of the invention is limited only by the scope of the appended claims.

## Claims

1. A method for purifying α-1 proteinase inhibitor from an aqueous solution containing α-1 proteinase inhibitor, comprising the steps of:
(a) removing a portion of contaminating proteins from the aqueous solution to obtain a purified solution containing α-1 proteinase inhibitor, wherein said removing step comprises the steps of
(i) precipitating said portion of contaminating proteins from said aqueous solution by adding a polyethylene glycol with a MW between about 3,000 and about 4000 to said aqueous solution at a concentration between about 3% to 15% weight per volume, and adjusting the pH of said aqueous solution from about 5.0 to about 6.0; and
(ii) separating said precipitated portion of contaminating proteins from said aqueous solution, thereby obtaining said purified solution containing α-1 proteinase inhibitor; then
(b) inactivating viruses prior to diluting said purified solution to reduce the conductivity of said purified solution so that α-1 proteinase inhibitor binds to an anion exchange resin, wherein said viral inactivation step comprises the steps of (a) adding a detergent to said purified solution to obtain a mixture of detergent and purified solution; and (b) adjusting the pH of said mixture to from about 6.5 to about 8.5, and wherein said detergent is a non-ionic detergent, and wherein said non-ionic detergent is Tween 20; then
(c) passing said purified solution through the anion exchange resin so that α-1 proteinase inhibitor binds to said anion exchange resin; then
(d) eluting α-1 proteinase inhibitor from said anion exchange resin to obtain an eluted solution containing α-1 proteinase inhibitor; then
(e) adjusting the pH, conductivity and protein concentration of said eluted solution containing α-1 proteinase inhibitor so that α-1 proteinase inhibitor does not bind to a cation exchange resin;
(f) passing the eluted solution through a cation exchange resin; and
(g) collecting a flow-through from said cation exchange resin that contains α-1 proteinase inhibitor.

2. A method for purifying α-1 proteinase inhibitor from Cohn Fraction IV-1, comprising the steps of;
(a) removing a portion of contaminating proteins from the Cohn Fraction IV-1 in order to obtain a purified solution containing α-1 proteinase inhibitor, wherein said removing step comprises the steps of
(i) precipitating said portion of contaminating proteins from said Cohn Fraction IV-1 by adding a polyethylene glycol with a MW between about 3,000 and about 4000 to said Cohn Fraction IV-1 at a concentration between about 3% to 15% weight per volume, and adjusting the pH of said Cohn Fraction IV-1 from about 5.0 to about 6.0; and
(ii) separating said precipitated portion of contaminating proteins from said Cohn Fraction IV-1, thereby obtaining said purified solution containing α-1 proteinase inhibitor; then
(b) inactivating viruses prior to adjusting the conductivity of said purified solution so that α-1 proteinase inhibitor binds to an anion exchange resin, wherein said viral inactivation step comprises the steps of (a) adding a detergent to said purified solution to obtain a mixture of detergent and purified solution; and (b) adjusting the pH of said mixture to from about 6.5 to about 8.5, and wherein said detergent is a non-ionic detergent, and wherein said non-ionic detergent is Tween 20; then
(c) passing said purified solution through the anion exchange resin so that α-1 proteinase inhibitor binds to said anion exchange resin; and then
(d) eluting α-1 proteinase inhibitor from said anion exchange resin to obtain an eluted solution containing α-1 proteinase inhibitor; then
(e) adjusting the pH, conductivity and protein concentration of said eluted solution containing α-1 proteinase inhibitor so that α-1 proteinase inhibitor does not bind to a cation exchange resin;
(f) passing the eluted solution through a cation exchange resin; and
(g) collecting a flow-through from said cation exchange resin that contains α-1 proteinase inhibitor.

3. The method of claim 1 or 2, further comprising the step of, prior to said eluting step, washing said anion exchange resin with a buffer solution to remove a portion of contaminating proteins from said anion exchange resin so that α-1 proteinase inhibitor remains bound to said anion exchange resin.

4. The method of claim 1 or 2, further comprising the step of removing viruses from said aqueous solution.

5. The method of claim 4, wherein said viral removal step comprises filtering said aqueous solution by nanofiltration.

6. The method of claim 1 or 2, wherein said diluting step (b) of claim 1 or said adjusting step (b) of claim 2 comprises diluting said purified solution so that said purified solution has a conductivity of between about 2.0 mS and about 6.0 mS.

7. The method of claim 6, wherein said purified solution is diluted with water.

8. The method of claim 7, wherein the water contains sodium phosphate.

9. The method of claim 1 or 2, further comprising the step of adjusting a pH of said purified solution to between about 6.25 and about 7.25 prior to passing said purified solution through the anion exchange resin.

10. The method of claim 2, wherein the Cohn Fraction IV-1 is suspended in an aqueous solution.

## Patentansprüche

1. Verfahren zur Aufreinigung von α-1 Proteinaseinhibitor aus einer wässrigen Lösung, die α-1 Proteinaseinhibitor enthält, umfassend die Schritte von:
(a) Entfernen von einem Teil von kontaminierenden Proteinen aus der wässrigen Lösung, um eine gereinigte Lösung zu erhalten, die α-1 Proteinaseinhibitor enthält, wobei der Schritt des Entfernens die Schritte umfasst:
(i) Präzipitieren von dem Teil der kontaminierenden Proteine aus der wässrigen Lösung durch die Zugabe eines Polyethylenglykols mit einem MW zwischen etwa 3.000 und etwa 4000 zu der wässrigen Lösung mit einer Konzentration zwischen etwa 3% bis 15 % Gewicht pro Volumen und das Einstellen von dem pH von der wässrigen Lösung auf etwa 5,0 bis etwa 6,0; und
(ii) Abtrennen des präzipitierten Teils der kontaminierenden Proteine von der wässrigen Lösung, wodurch die gereinigte Lösung erhalten wird, die α-1 Proteinaseinhibitor enthält; dann
(b) Inaktivieren von Viren vor dem Verdünnen der gereinigten Lösung, um die Leitfähigkeit von der gereinigten Lösung zu verringern, sodass der α-1 Proteinaseinhibitor an ein Anionenaustauscherharz bindet, wobei der Schritt des Virusinaktivierens die Schritte (a) Zugeben von einem Detergenz zu der gereinigten Lösung, um eine Mischung von Detergenz und gereinigter Lösung zu erhalten; und (b) Einstellen von dem pH von der Mischung auf etwa 6,5 bis etwa 8,5 umfasst, und wobei das Detergenz ein nichtionisches Detergenz ist, und wobei das nichtionische Detergenz Tween 20 ist; dann
(c) Durchlaufen-Lassen von der gereinigten Lösung durch das Anionenaustauscherharz, sodass der α-1 Proteinaseinhibitor an das Anionenaustauscherharz bindet; dann
(d) Eluieren von α-1 Proteinaseinhibitor aus dem Anionenaustauscherharz, um eine eluierte Lösung zu erhalten, die α-1 Proteinaseinhibitor enthält; dann
(e) Einstellen von dem pH, der Leitfähigkeit und der Proteinkonzentration von der eluierten Lösung, die α-1 Proteinaseinhibitor enthält, sodass der α-1 Proteinaseinhibitor nicht an ein Kationenaustauscherharz bindet;
(f) Durchlaufen-Lassen von der eluierten Lösung durch ein Kationenaustauscherharz; und
(g) Sammeln eines Durchlaufs von dem Kationenaustauscherharz, der α-1 Proteinaseinhibitor enthält.

2. Verfahren zur Aufreinigung von α-1 Proteinaseinhibitor aus einer Cohn-Fraktion IV-1, umfassend die Schritte von:
(a) Entfernen von einem Teil von kontaminierenden Proteinen aus der Cohn-Fraktion IV-1, um eine gereinigte Lösung zu erhalten, die α-1 Proteinaseinhibitor enthält, wobei der Schritt des Entfernens die Schritte umfasst:
(i) Präzipitieren von dem Teil der kontaminierenden Proteine aus der Cohn-Fraktion IV-1 durch die Zugabe eines Polyethylenglykols mit einem MW zwischen etwa 3.000 und etwa 4000 zu der Cohn-Fraktion IV-1 mit einer Konzentration zwischen etwa 3% bis 15 % Gewicht pro Volumen und das Einstellen von dem pH von der Cohn-Fraktion IV-1 auf etwa 5,0 bis etwa 6,0; und
(ii) Abtrennen des präzipitierten Teils der kontaminierenden Proteine von der Cohn-Fraktion IV-1, wodurch die gereinigte Lösung erhalten wird, die α-1 Proteinaseinhibitor enthält; dann
(b) Inaktivieren von Viren vor dem Einstellen der Leitfähigkeit von der gereinigten Lösung, sodass der α-1 Proteinaseinhibitor an ein Anionenaustauscherharz bindet, wobei der Schritt des Virusinaktivierens die Schritte (a) Zugeben von einem Detergenz zu der gereinigten Lösung, um eine Mischung von Detergenz und gereinigter Lösung zu erhalten; und (b) Einstellen von dem pH von der Mischung auf etwa 6,5 bis etwa 8,5 umfasst, und wobei das Detergenz ein nichtionisches Detergenz ist, und wobei das nichtionische Detergenz Tween 20 ist; dann
(c) Durchlaufen-Lassen von der gereinigten Lösung durch das Anionenaustauscherharz, sodass der α-1 Proteinaseinhibitor an das Anionenaustauscherharz bindet; dann
(d) Eluieren von α-1 Proteinaseinhibitor aus dem Anionenaustauscherharz, um eine eluierte Lösung zu erhalten, die α-1 Proteinaseinhibitor enthält; dann
(e) Einstellen von dem pH, der Leitfähigkeit und der Proteinkonzentration von der eluierten Lösung, die α-1 Proteinaseinhibitor enthält, sodass der α-1 Proteinaseinhibitor nicht an ein Kationenaustauscherharz bindet;
(f) Durchlaufen-Lassen von der eluierten Lösung durch ein Kationenaustauscherharz; und
(g) Sammeln eines Durchlaufs von dem Kationenaustauscherharz, der α-1 Proteinaseinhibitor enthält.

3. Verfahren nach Anspruch 1 oder 2, ferner umfassend vor dem Schritt des Eluierens den Schritt des Waschens von dem Anionenaustauscherharz mit einer Pufferlösung, sodass der α-1 Proteinaseinhibitor an das Anionenaustauscherharz gebunden bleibt, um einen Teil der kontaminierenden Proteine aus dem Anionenaustauscherharz zu entfernen.

4. Verfahren nach Anspruch 1 oder 2, ferner umfassend den Schritt des Entfernens von Viren aus der wässerigen Lösung.

5. Verfahren nach Anspruch 4, wobei der Schritt des Virusentfernens das Filtern der wässrigen Lösung mittels Nanofiltration umfasst.

6. Verfahren nach Anspruch 1 oder 2, wobei der Schritt des Einstellens das Verdünnen von der gereinigten Lösung umfasst, sodass die gereinigte Lösung eine Leitfähigkeit von zwischen etwa 2,0 mS und etwa 6,0 mS hat.

7. Verfahren nach Anspruch 6, wobei die gereinigte Lösung mit Wasser verdünnt wird.

8. Verfahren nach Anspruch 7, wobei das Wasser Natriumphosphat enthält.

9. Verfahren nach Anspruch 1 oder 2, ferner umfassend vor dem Schritt des Durchlaufen-Lassens von der gereinigten Lösung durch das Anionenaustauscherharz den Schritt des Einstellens von einem pH von der gereinigten Lösung auf zwischen etwa 6,25 und etwa 7,25.

10. Verfahren nach Anspruch 2, wobei die Cohn-Fraktion IV-1 in einer wässrigen Lösung suspendiert ist.

## Revendications

1. Procédé pour purifier un inhibiteur de l'α-1 protéinase à partir d'une solution aqueuse contenant l'inhibiteur de l'α-1 protéinase, comprenant les étapes consistant à :
(a) retirer de la solution aqueuse une portion des protéines contaminantes pour obtenir une solution purifiée contenant l'inhibiteur de l'α-1 protéinase, dans lequel ladite étape de retrait comprend les étapes consistant à :
(i) précipiter ladite portion de protéines contaminantes de ladite solution aqueuse en ajoutant à ladite solution aqueuse un polyéthylène glycol ayant un poids moléculaire entre environ 3000 et 4000 à une concentration entre environ 3% à 15% en poids par volume, et en ajustant le pH de ladite solution aqueuse d'environ 5.0 à environ 6.0 ; et
(ii) séparer ladite portion de protéines contaminantes précipitée de ladite solution aqueuse, afin d'obtenir ladite solution purifiée contenant l'inhibiteur de l'α-1 protéinase ; ensuite
(b) inactiver les virus avant de diluer ladite solution purifiée pour réduire la conductivité de ladite solution purifiée de façon que l'inhibiteur de l'α-1 protéinase se lie à une résine échangeuse d'anions, dans laquelle l'étape d'inactivation des virus comprend les étapes de (a) ajouter un détergent à ladite solution purifiée pour obtenir un mélange de détergent et de solution purifiée ; et (b) ajuster le pH dudit mélange d'environ 6.5 à environ 8.5, et dans laquelle ledit détergent est un détergent non-ionique, et dans laquelle ledit détergent non-ionique est du Tween 20 ; ensuite
(c) faire passer ladite solution à travers une résine échangeuse d'anions afin que l'inhibiteur de l'α-1 protéinase se lie à ladite résine échangeuse d'anions ; ensuite
(d) éluer l'inhibiteur de l'α-1 protéinase à partir de ladite résine échangeuse d'anions pour obtenir une solution éluée contenant l'inhibiteur de l'α-1 protéinase ; ensuite
(e) ajuster le pH, la conductivité et la concentration de protéines de ladite solution éluée contenant l'inhibiteur de l'α-1 protéinase de façon que l'inhibiteur de l'α-1 protéinase ne se lie pas à une résine échangeuse de cations ;
(f) faire passer la solution éluée à travers une résine échangeuse de cations ; et
(g) collecter un effluent traversant et provenant de ladite résine échangeuse de cations, qui contient l'inhibiteur de l'α-1 protéinase.

2. Procédé pour purifier un inhibiteur de l'α-1 protéinase à partir d'une Fraction IV-1 de Cohn, comprenant les étapes consistant à :
(a) retirer de la Fraction IV-1 de Cohn une portion des protéines contaminantes dans le but d'obtenir une solution purifiée contenant l'inhibiteur de l'α-1 protéinase, dans lequel ladite étape de retrait comprend les étapes consistant à :
(i) précipiter ladite portion de protéines contaminantes de ladite Fraction IV-1 de Cohn en ajoutant à ladite Fraction IV-1 de Cohn un polyéthylène glycol ayant un poids moléculaire entre environ 3000 et 4000 à une concentration entre environ 3% à 15% en poids par volume, et en ajustant le pH de ladite Fraction IV-1 de Cohn d'environ 5.0 à environ 6.0; et
(ii) séparer ladite portion de protéines contaminantes précipitée de ladite Fraction IV-1 de Cohn, afin d'obtenir ladite solution purifiée contenant l'inhibiteur de l'α-1 protéinase ; ensuite
(b) inactiver les virus avant de diluer ladite solution purifiée pour réduire la conductivité de ladite solution purifiée de façon que l'inhibiteur de l'α-1 protéinase se lie à une résine échangeuse d'anions, dans laquelle l'étape d'inactivation des virus comprend les étapes de (a) ajouter un détergent à ladite solution purifiée pour obtenir un mélange de détergent et de solution purifiée ; et (b) ajuster le pH dudit mélange d'environ 6.5 à environ 8.5, et dans laquelle ledit détergent est un détergent non-ionique, et dans laquelle ledit détergent non-ionique est du Tween 20; ensuite
(c) faire passer ladite solution purifiée à travers une résine échangeuse d'anions de sorte que l'inhibiteur de l'α-1 protéinase se lie à ladite résine échangeuse d'anions ; et ensuite
(d) éluer l'inhibiteur de l'α-1 protéinase à partir de ladite résine échangeuse d'anions pour obtenir une solution éluée contenant l'inhibiteur de l'α-1 protéinase ; ensuite
(e) ajuster le pH, la conductivité et la concentration de protéines de ladite solution éluée contenant l'inhibiteur de l'α-1 protéinase de façon que l'inhibiteur de l'α-1 protéinase ne se lie pas à une résine échangeuse de cations ;
(f) faire passer la solution éluée à travers une résine échangeuse de cations ; et
(g) collecter un effluent traversant et provenant de ladite résine échangeuse de cations qui contient l'inhibiteur de l'α-1 protéinase.

3. Procédé selon la revendication 1 ou 2, comprenant en outre l'étape consistant à, préalablement à ladite étape d'élution, laver ladite résine échangeuse d'anions avec une solution tampon pour extraire une portion de protéines contaminantes de ladite résine échangeuse d'anions de sorte que l'inhibiteur de l'α-1 protéinase reste lié à ladite résine échangeuse d'anions.

4. Procédé selon la revendication 1 ou 2, comprenant en outre l'étape consistant à retirer les virus de ladite solution aqueuse.

5. Procédé selon la revendication 4, dans lequel l'étape de retrait des virus comprend le filtrage de ladite solution aqueuse par nano filtration.

6. Procédé selon la revendication 1 ou 2, dans lequel ladite étape de dilution (b) de la revendication 1 ou ladite étape d'ajustement (b) de la revendication 2 comprend la dilution de ladite solution purifiée de façon que ladite solution purifiée ait une conductivité entre environ 2.0 mS et environ 6.0 mS.

7. Procédé selon la revendication 6, dans lequel ladite solution purifiée est diluée avec de l'eau.

8. Procédé selon la revendication 7, dans lequel l'eau contient du phosphate de sodium.

9. Procédé selon la revendication 1 ou 2, comprenant en outre l'étape consistant à ajuster un pH de ladite solution purifiée entre environ 6.25 et environ 7.25 avant de faire passer ladite solution purifiée à travers la résine échangeuse d'anions.

10. Procédé selon la revendication 2, dans lequel la Fraction IV-1 de Cohn est mise en suspension dans une solution aqueuse.
